# EUROPEAN PATENT SPECIFICATION

(11) **EP 0 764 204 B1**
(45) Date of publication and mention of the grant of the patent: **22.08.2001**
(21) Application number: 94924439.6
(22) Date of filing: 18.05.1994
(51) Int. Cl.: C12N 9/88, C12N 15/60, A61K 38/43

(54) **HUMAN OXALYL-CoA DECARBOXYLASE**
HUMANE OXALYL-CoA DECARBOXYLASE
OXALYL-CoA DECARBOXYLASE HUMAINE

(43) Date of publication of application: 26.03.1997
(73) Proprietor: HUMAN GENOME SCIENCES, INC., Rockville, MD 20850-3338 (US)
(72) Inventor: OLSEN, Henrik, Gaithersburg, MD 20878 (US); COLEMAN, Timothy A., Gaithersburg, MD 20878 (US); ADAMS, Mark D., North Potomac, MD 20878 (US)
(74) Representative: VOSSIUS & PARTNER
(86) International application number: US9405561
(87) International publication number: WO9531537

(56) References cited:
- FIle Medline, abstract 91181624, 1991 XP002035813 & AM. J. KIDNEY DISEASES, vol. 17, no. 4, April 1991, pages 381-385, H Y LUNG ET AL.: "Cloning and expression of the oxalyl-CoA decarboxylase gene from the bacterium, Oxalobacter formigenes; prospects for gene therapy to control Ca-oxalate kidney stone formation"
- BIOCHEM. J., Volume 118, issued 1970, M.A. BLACKMORE et al., "Microbial Growth on Oxalate by a Route Not Involving Glyoxylate Carboligase", pages 53-59.
- J. BACTERIOL., Volume 171, No. 5, issued May 1989, A.L. BAETZ et al., "Purification and Characterization of Oxalyl-Coenzyme A Decarboxylase From Oxalobacter Formigenes", pages 2605-2608.
- J. BACTERIOL., Volume 176, No. 8, issued April 1994, LUNG et al., "Molecular Cloning, DNA Sequence and Gene Expression of the Oxalyl-Coenzyme A Decarboxylase Gene, Oxc, From the Bacterium Oxalobacter Formigenes", pages 2468-2472.
- CHEMICAL ABSTRACTS, Volume 118, issued 1993, A.L. BAETZ et al., "Localization of Oxalyl-Coenzyme a Decarboxylase and Formyl-Coenzyme A Transferase in Oxalobacter Formigenes Cells", page 406, Abstract No. 143063p; & SYST. APPL. MICROBIOL., 15(2), pages 167-171.

## Description

This invention relates to newly identified polynucleotides, polypeptides encoded by such polynucleotides, the use of such polynucleotides and polypeptides, as well as the production of such polynucleotides and polypeptides. More particularly, the polypeptide of the present invention is human Oxalyl-CoA Decarboxylase.

Evidence suggests that the formation of calcium-oxalate stones in the urine is dependent on the saturation levels of both calcium and oxalate, thus, management of one or both of these ions in individuals susceptible to stone formation in the urinary tract (urolithiasis) appears important. Urolithiasis is a common urinary tract problem afflicting more than 10% of the U.S. population (Sierakowski, R. et al., Invest. Urol., 15:438-441 (1978)). Urinary tract stones are usually classified according to their composition, with the most frequently encountered (70%) being the calcium stone which is composed of calcium oxalate alone or calcium oxalate mixed with calcium phosphate. Although precipitation of calcium oxalate depends on a urine saturated with both calcium and oxalate ions in a meta-stable state, it has been argued that the oxalate ion concentration is more significant in the formation of urinary calcium oxalate stones.

The majority of oxalate in plasma and urine is derived from the endogenous metabolism of ascorbic acid, glyoxylate, and to a lesser degree, tryptophan (Nath, R. et al., Pergamon Press, pp. 55-58 (1984)). In addition, between 10% and 20% of the urinary oxalate is absorbed from the diet, especially through ingestion of leafy vegetables and plant materials. Fortunately, most dietary oxalate appears to be bound by intraluminal calcium and is excreted as an insoluble salt. Thus, there is an inverse relationship between ingested calcium and absorbed oxalate. (Ernest, D.L., et al., Gastroenterology, 66:1114-1122 (1964)).

Either abnormal synthesis or hyper-absorption of oxalate can lead to a serious condition referred to as hyperoxaluria (Liedtke, R.R. et al., Urol. Res., 16:188-189 (1988)). Although this condition may have a genetic basis, the vast majority of cases remain idiopathic (Nath, R. et al., Pergamon Press, pp. 55-58 (1984)). Whether the underlying cause is a disturbance in calcium metabolism or merely increased levels of oxalate there is a strong association between increased levels of urinary oxalate and calcium oxalate stone disease in man.

The basis of stone formation in the urinary tract and ways to treat this disorder has recently been the subject of intensive study. A plant-derived oxalyl-CoA decarboxylase gene has been inserted into human cells as a means of lowering plasma and urinary oxalate concentrations. The oxalyl-CoA decarboxylase gene has been cloned from bacterium *Oxalobacter formigenes*. Lung, H.Y. et al., Am. J. Kidney Dis., 17:381-5 (1991).

Accordingly, an enzyme that lowers the oxalate levels in the plasma, and subsequently the urine, would decrease the incidence of calcium oxalate stone formation.

In accordance with one aspect of the present invention, there is provided a novel mature polypeptide which is human Oxalyl-CoA Decarboxylase.

In accordance with another aspect of the present invention, there are provided polynucleotides (DNA or RNA) which encode such polypeptides.

In accordance with yet a further aspect of the present invention, there is provided a process for producing such polypeptide by recombinant techniques.

In accordance with yet a further aspect of the present invention, there is provided a process for utilizing such polypeptide, or polynucleotide encoding such polypeptide for in the preparation of medicaments useful, for example, in preventing calcium-oxalate stone formation and hyperoxaluria.

In accordance with yet a further aspect of the present invention, there is provided an antibody against such polypeptides.

These and other aspects of the present invention should be apparent to those skilled in the art from the teachings herein.

The following drawings are illustrative of embodiments of the invention.

FIG. 1 displays the cDNA sequence and corresponding deduced amino acid sequence of the mature oxalyl-CoA decarboxylase polypeptide. The standard three letter abbreviation for amino acids is used.

FIG. 2 is an amino acid sequence comparison between oxalyl-CoA decarboxylase from the bacterium *Oxalobacter formigenes* (upper line) and the polypeptide encoded by the polynucleotide sequence of the present invention (lower line).

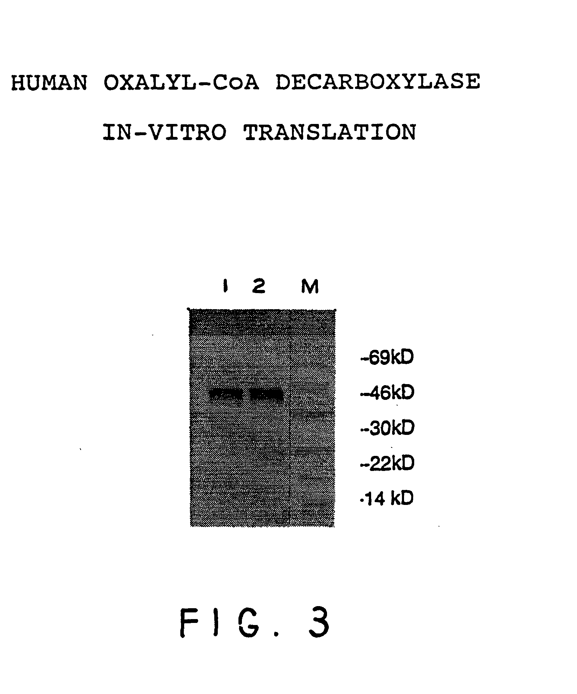

FIG. 3 shows the results of electrophoresing human oxalyl-CoA decarboxylase on a gel after *in vitro* transcription/translation. Lane 1 is a plasmid template encoding the human oxalyl-CoA decarboxylase. Lane 2 is a PCR generated template encoding the human oxalyl-CoA decarboxylase, and M stands for molecular weight marker.

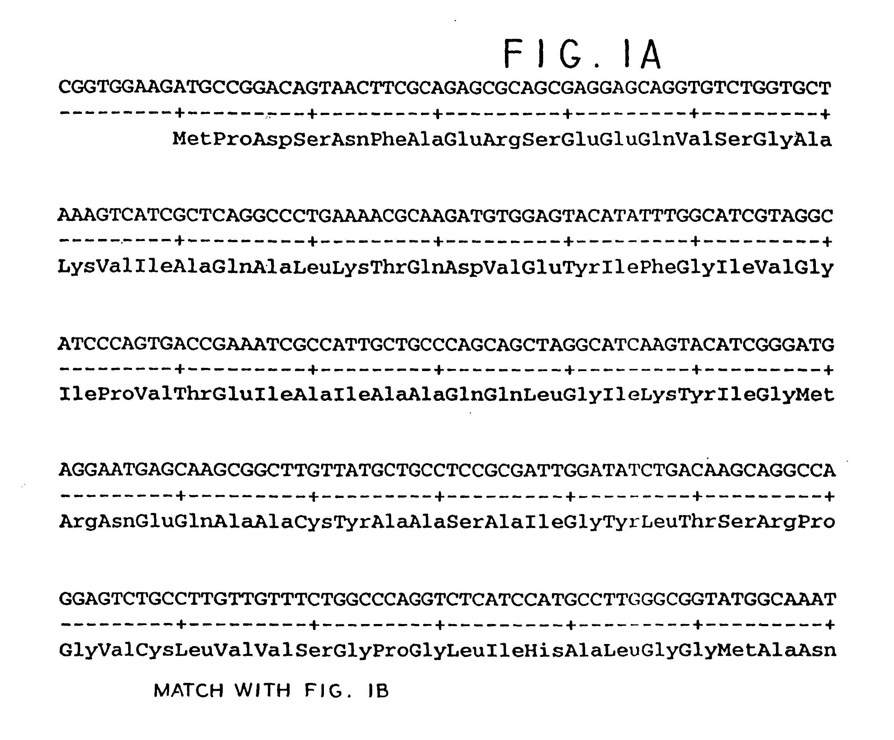

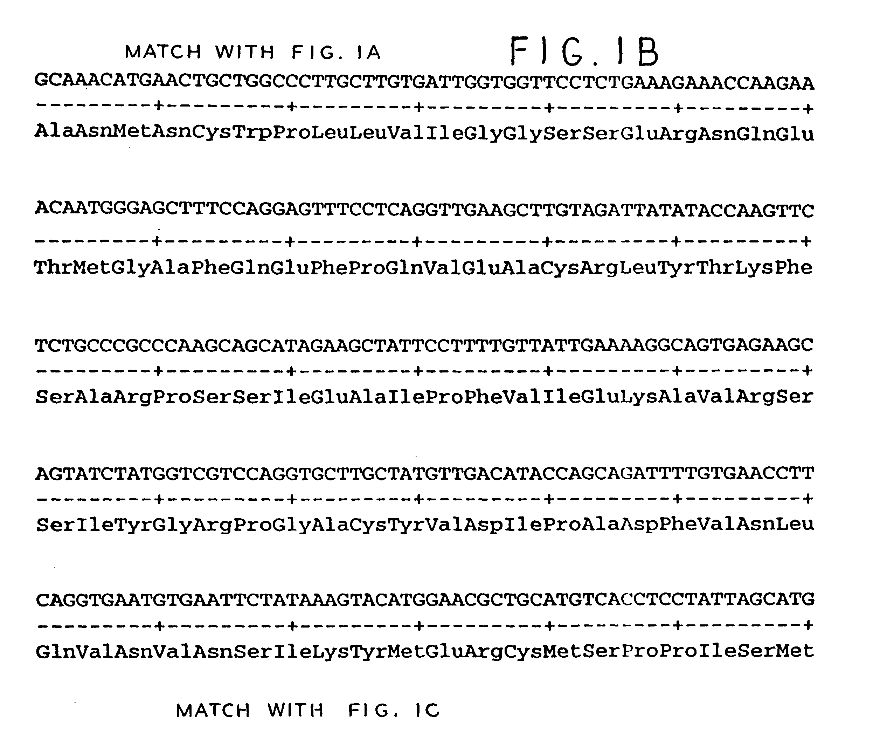

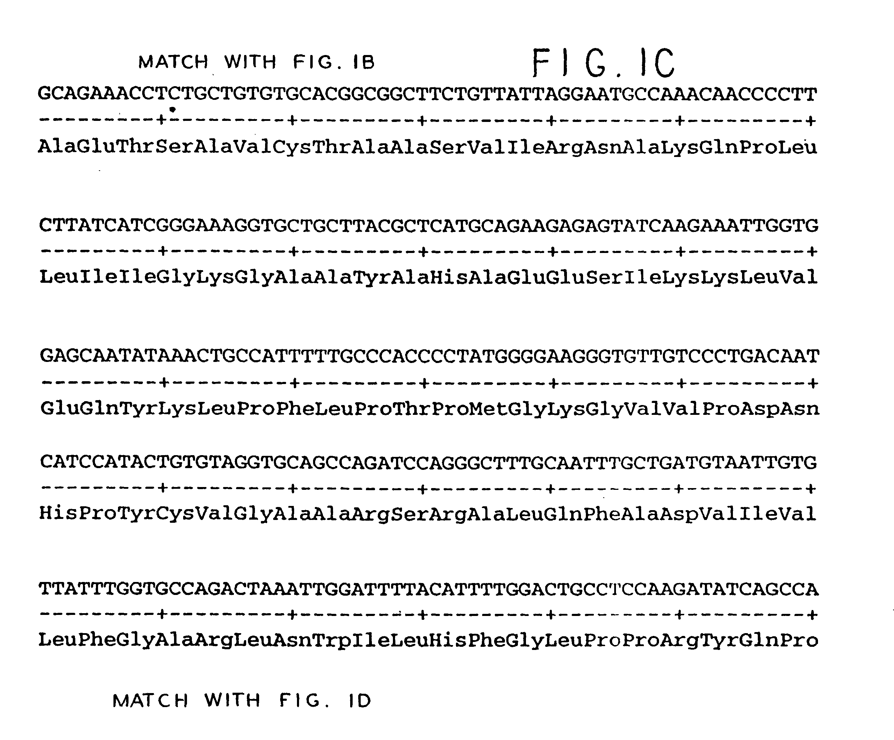

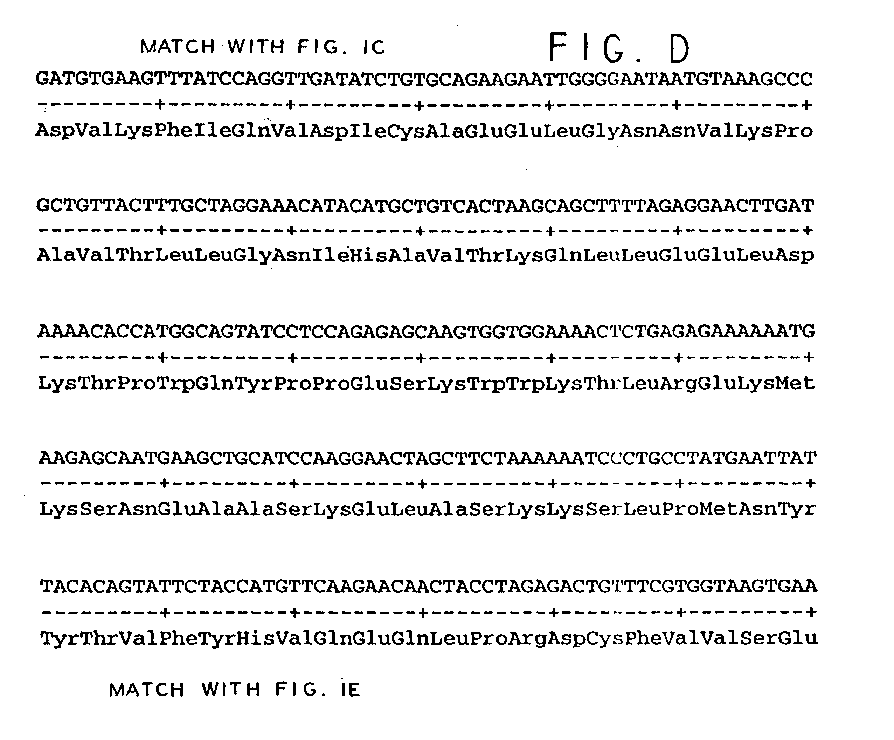

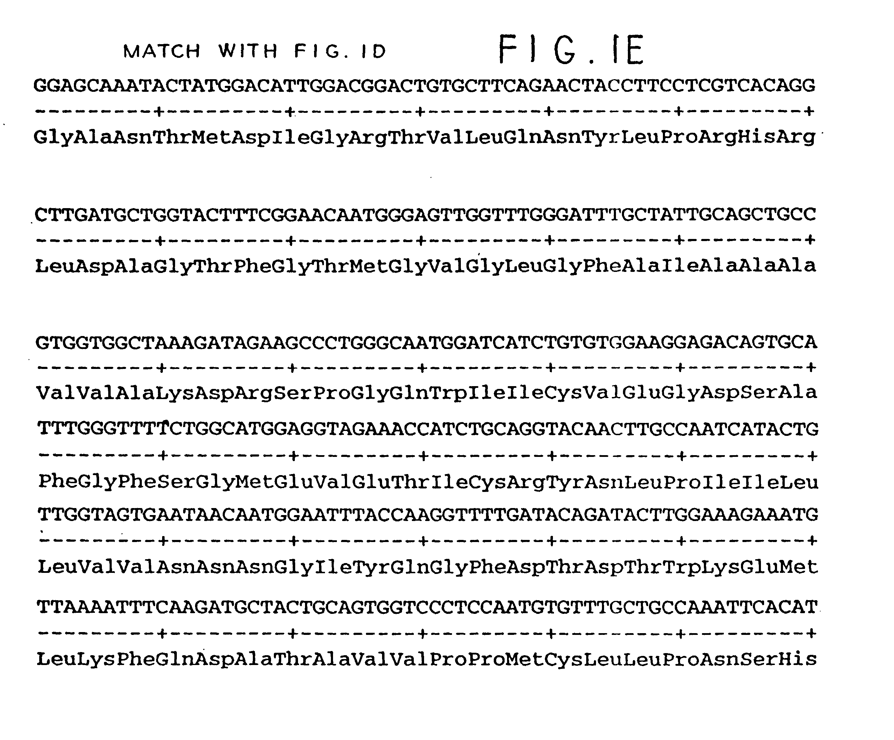

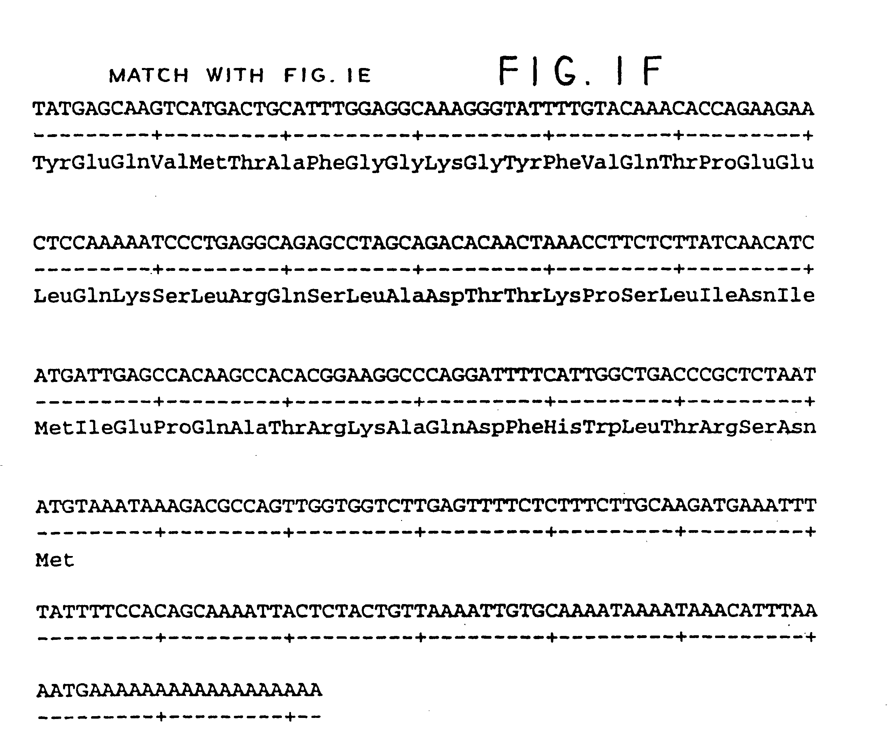

In accordance with an aspect of the present invention, there is provided an isolated nucleic acid (polynucleotide) which encodes for the mature polypeptide having the deduced amino acid sequence of Figure 1 or for the mature polypeptide encoded by the cDNA of the clone deposited as ATCC Deposit No. 75715 on March, 18, 1994.

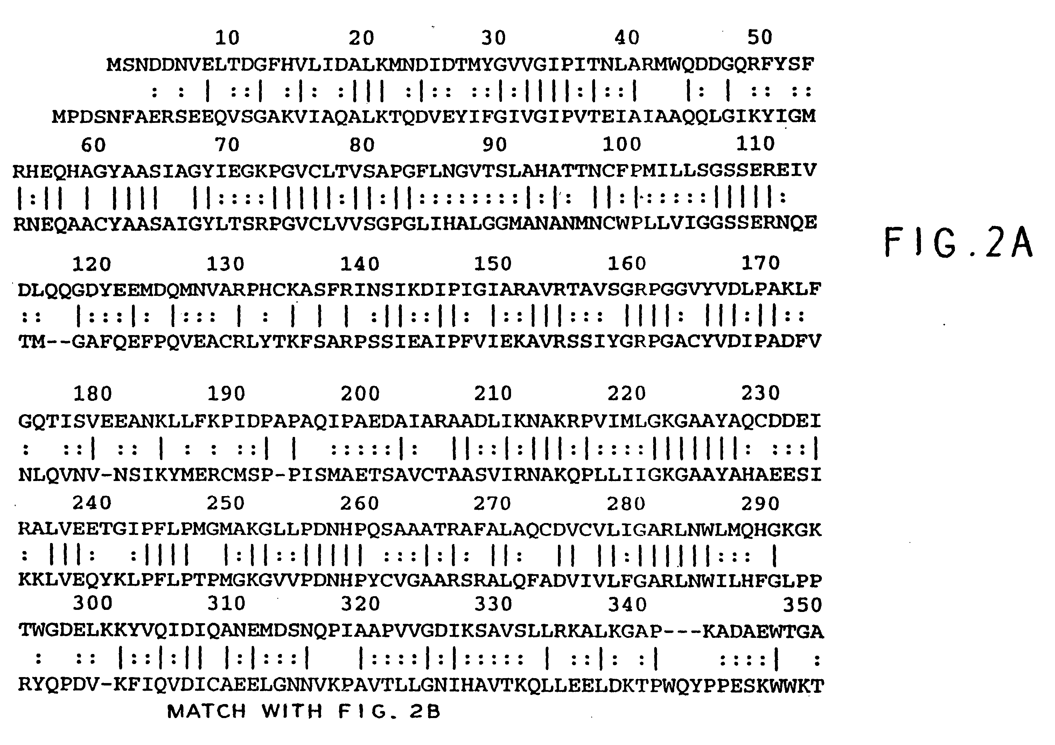

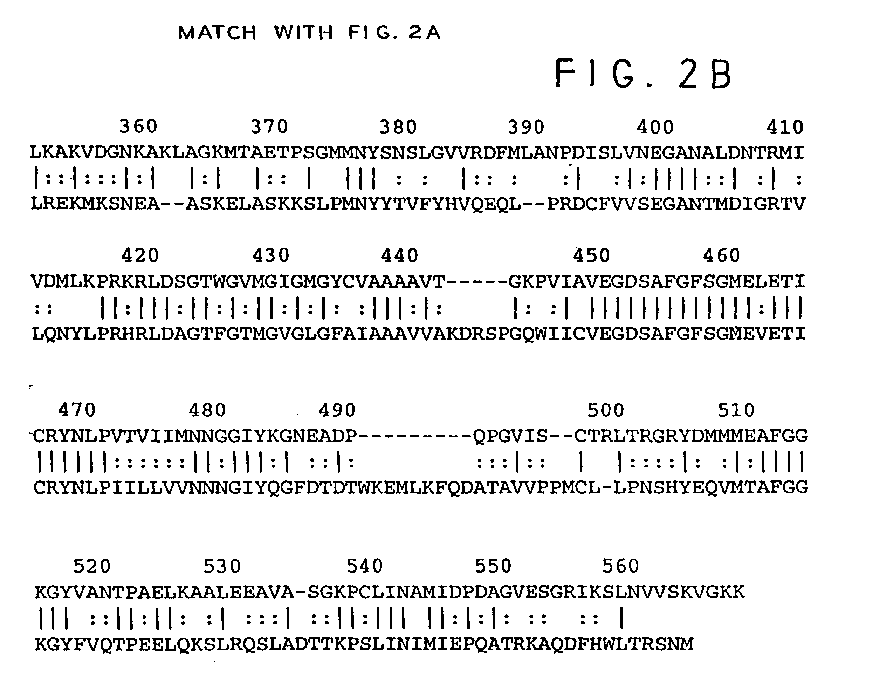

The polynucleotide of this invention was discovered in a cDNA library derived from the human pancreas. It contains an open reading frame encoding a mature protein of 578 amino acid residues. The protein of the present invention is approximately 50-60% homologous to the oxalyl-CoA Decarboxylase from the bacterium *Oxalobacter formigenes* at the amino acid level. The homology starts at amino acid 8 of the bacterial enzyme (see Figure 2).

The polynucleotide of the present invention may be in the form of RNA or in the form of DNA, which DNA includes cDNA, genomic DNA, and synthetic DNA. The DNA may be doublestranded or single-stranded, and if single stranded may be the coding strand or non-coding (anti-sense) strand. The coding sequence which encodes the mature polypeptide may be identical to the coding sequence shown in Figure 1 or that of the deposited clone or may be a different coding sequence which coding sequence, as a result of the redundancy or degeneracy of the genetic code, encodes the same, mature polypeptide as the DNA of Figure 1 or the deposited cDNA.

The polynucleotide which encodes for the mature polypeptide of Figure 1 or for the mature polypeptide encoded by the deposited cDNA may include: only the coding sequence for the mature polypeptide; the coding sequence for the mature polypeptide and additional coding sequence such as a leader or secretory sequence or a proprotein sequence; the coding sequence for the mature polypeptide (and optionally additional coding sequence) and non-coding sequence, such as introns or non-coding sequence 5' and/or 3' of the coding sequence for the mature polypeptide.

Thus, the term "polynucleotide encoding a polypeptide" encompasses a polynucleotide which includes only coding sequence for the polypeptide as well as a polynucleotide which includes additional coding and/or non-coding sequence.

The present invention further relates to variants of the hereinabove described polynucleotides which encode for fragments, analogs and derivatives of the polypeptide having the deduced amino acid sequence of Figure 1 or the polypeptide encoded by the cDNA of the deposited clone. The variant of the polynucleotide may be a naturally occurring allelic variant of the polynucleotide or a non-naturally occurring variant of the polynucleotide.

Thus, the present invention includes polynucleotides encoding the same mature polypeptide as shown in Figure 1 or the same mature polypeptide encoded by the cDNA of the deposited clone as well as variants of such polynucleotides which variants encode for a fragment, derivative or analog of the polypeptide of Figure 1 or the polypeptide encoded by the cDNA of the deposited clone. Such nucleotide variants include deletion variants, substitution variants and addition or insertion variants.

As hereinabove indicated, the polynucleotide may have a coding sequence which is a naturally occurring allelic variant of the coding sequence shown in Figure 1 or of the coding sequence of the deposited clone. As known in the art, an allelic variant is an alternate form of a polynucleotide sequence which may have a substitution, deletion or addition of one or more nucleotides, which does not substantially alter the function of the encoded polypeptide.

The polynucleotides of the present invention may also have the coding sequence fused in frame to a marker sequence which allows for purification of the polypeptide of the present invention. The marker sequence may be a hexahistidine tag supplied by a pQE-9 vector to provide for purification of the mature polypeptide fused to the marker in the case of a bacterial host, or, for example, the marker sequence may be a hemagglutinin (HA) tag when a mammalian host, e.g. COS-7 cells, is used. The HA tag corresponds to an epitope derived from the influenza hemagglutinin protein (Wilson, I., et al., Cell, 37:767 (1984)).

The present invention further relates to polynucleotides which hybridize to the hereinabove-described sequences if there is at least 70% identity between the sequences. The present invention particularly relates to polynucleotides which hybridize under stringent conditions to the hereinabove-described polynucleotides . As herein used, the term "stringent conditions" means hybridization will occur only if there is at least 95% and preferably at least 97% identity between the sequences. The polynucleotides which hybridize to the hereinabove described polynucleotides in a preferred embodiment encode polypeptides which retain substantially the same biological function or activity as the mature polypeptide encoded by the cDNA of Figure 1 or the deposited cDNA.

The deposit(s) referred to herein will be maintained under the terms of the Budapest Treaty on the International Recognition of the Deposit of Micro-organisms for purposes of Patent Procedure.

The present invention further relates to an Oxalyl-CoA Decarboxylase polypeptide which has the deduced amino acid sequence of Figure 1 or which has the amino acid sequence encoded by the deposited cDNA, as well as fragments, analogs and derivatives of such polypeptide.

The terms "fragment," "derivative" and "analog" when referring to the polypeptide of Figure 1 or that encoded by the deposited cDNA, means a polypeptide which retains essentially the same biological function or activity as such polypeptide. Thus, an analog includes a proprotein which can be activated by cleavage of the proprotein portion to produce an active mature polypeptide.

The polypeptide of the present invention may be a recombinant polypeptide, a natural polypeptide or a synthetic polypeptide, preferably a recombinant polypeptide.

The fragment, derivative or analog of the polypeptide of Figure 1 or that encoded by the deposited cDNA may be (i) one in which one or more of the amino acid residues are substituted with a conserved or non-conserved amino acid residue (preferably a conserved amino acid residue) and such substituted amino acid residue may or may not be one encoded by the genetic code, or (ii) one in which one or more of the amino acid residues includes a substituent group, or (iii) one in which the mature polypeptide is fused with another compound, such as a compound to increase the half-life of the polypeptide (for example, polyethylene glycol), or (iv) one in which the additional amino acids are fused to the mature polypeptide, such as a leader or secretory sequence or a sequence which is employed for purification of the mature polypeptide or a proprotein sequence. Such fragments, derivatives and analogs are deemed to be within the scope of those skilled in the art from the teachings herein.

The polypeptides and polynucleotides of the present invention are preferably provided in an isolated form, and preferably are purified to homogeneity.

The term "isolated" means that the material is removed from its original environment (e.g., the natural environment if it is naturally occurring). For example, a naturally-occurring polynucleotide or polypeptide present in a living animal is not isolated, but the same polynucleotide or polypeptide, separated from some or all of the coexisting materials in the natural system, is isolated. Such polynucleotides could be part of a vector and/or such polynucleotides or polypeptides could be part of a composition, and still be isolated in that such vector or composition is not part of its natural environment.

The present invention also relates to vectors which include polynucleotides of the present invention, host cells which are genetically engineered with vectors of the invention and the production of polypeptides of the invention by recombinant techniques.

Host cells are genetically engineered (transduced or transformed or transfected) with the vectors of this invention which may be, for example, a cloning vector or an expression vector. The vector may be, for example, in the form of a plasmid, a viral particle, a phage, etc. The engineered host cells can be cultured in conventional nutrient media modified as appropriate for activating promoters, selecting transformants or amplifying the Oxalyl-CoA Decarboxylase genes. The culture conditions, such as temperature, pH and the like, are those previously used with the host cell selected for expression, and will be apparent to the ordinarily skilled artisan.

The polynucleotides of the present invention may be employed for producing polypeptides by recombinant techniques. Thus, for example, the polynucleotide may be included in any one of a variety of expression vectors for expressing a polypeptide. Such vectors include chromosomal, nonchromosomal and synthetic DNA sequences, e.g., derivatives of SV40; bacterial plasmids; phage DNA; baculovirus; yeast plasmids; vectors derived from combinations of plasmids and phage DNA, viral DNA such as vaccinia, adenovirus, fowl pox virus, and pseudorabies. However, any other vector may be used as long as it is replicable and viable in the host.

The appropriate DNA sequence may be inserted into the vector by a variety of procedures. In general, the DNA sequence is inserted into an appropriate restriction endonuclease site(s) by procedures known in the art. Such procedures and others are deemed to be within the scope of those skilled in the art.

The DNA sequence in the expression vector is operatively linked to an appropriate expression control sequence(s) (promoter) to direct mRNA synthesis. As representative examples of such promoters, there may be mentioned: LTR or SV40 promoter, the E. coli. lac or trp, the phage lambda P_{L} promoter and other promoters known to control expression of genes in prokaryotic or eukaryotic cells or their viruses. The expression vector also contains a ribosome binding site for translation initiation and a transcription terminator. The vector may also include appropriate sequences for amplifying expression.

In addition, the expression vectors preferably contain one or more selectable marker genes to provide a phenotypic trait for selection of transformed host cells such as dihydrofolate reductase or neomycin resistance for eukaryotic cell culture, or such as tetracycline or ampicillin resistance in E. coli.

The vector containing the appropriate DNA sequence as hereinabove described, as well as an appropriate promoter or control sequence, may be employed to transform an appropriate host to permit the host to express the protein.

As representative examples of appropriate hosts, there may be mentioned: bacterial cells, such as E. coli, Streptomyces, Salmonella typhimurium; fungal cells, such as yeast; insect cells such as Drosophila and Sf9; animal cells such as CHO, COS or Bowes melanoma; plant cells, etc. The selection of an appropriate host is deemed to be within the scope of those skilled in the art from the teachings herein.

More particularly, the present invention also includes recombinant constructs comprising one or more of the sequences as broadly described above. The constructs comprise a vector, such as a plasmid or viral vector, into which a sequence of the invention has been inserted, in a forward or reverse orientation. In a preferred aspect of this embodiment, the construct further comprises regulatory sequences, including, for example, a promoter, operably linked to the sequence. Large numbers of suitable vectors and promoters are known to those of skill in the art, and are commercially available. The following vectors are provided by way of example. Bacterial: pQE70, pQE60, pQE-9 (Qiagen), pbs, pD10, phagescript, psiX174, pbluescript SK, pbsks, pNH8A, pNH16a, pNH18A, pNH46A (Stratagene); ptrc99a, pKK223-3, pKK233-3, pDR540, pRIT5 (Pharmacia). Eukaryotic: pWLNEO, pSV2CAT, pOG44, pXT1, pSG (Stratagene) pSVK3, pBPV, pMSG, pSVL (Pharmacia). However, any other plasmid or vector may be used as long as they are replicable and viable in the host.

Promoter regions can be selected from any desired gene using CAT (chloramphenicol transferase) vectors or other vectors with selectable markers. Two appropriate vectors are PKK232-8 and PCM7. Particular named bacterial promoters include lacI, lacZ, T3, T7, gpt, lambda P_{R}, P_{L} and trp. Eukaryotic promoters include CMV immediate early, HSV thymidine kinase, early and late SV40, LTRs from retrovirus, and mouse metallothionein-I. Selection of the appropriate vector and promoter is well within the level of ordinary skill in the art.

In a further embodiment, the present invention relates to host cells containing the above-described constructs. The host cell can be a higher eukaryotic cell, such as a mammalian cell, or a lower eukaryotic cell, such as a yeast cell, or the host cell can be a prokaryotic cell, such as a bacterial cell. Introduction of the construct into the host cell can be effected by calcium phosphate transfection, DEAE-Dextran mediated transfection, or electroporation. (Davis, L., Dibner, M., Battey, I., Basic Methods in Molecular Biology, (1986)).

The constructs in host cells can be used in a conventional manner to produce the gene product encoded by the recombinant sequence. Alternatively, the polypeptides of the invention can be synthetically produced by conventional peptide synthesizers.

Mature proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook, et al., Molecular Cloning: A Laboratory Manual, Second Edition, Cold Spring Harbor, N.Y., (1989).

Transcription of the DNA encoding the polypeptides of the present invention by higher eukaryotes is increased by inserting an enhancer sequence into the vector. Enhancers are cis-acting elements of DNA, usually about from 10 to 300 bp that act on a promoter to increase its transcription. Examples including the SV40 enhancer on the late side of the replication origin bp 100 to 270, a cytomegalovirus early promoter enhancer, the polyoma enhancer on the late side of the replication origin, and adenovirus enhancers.

Generally, recombinant expression vectors will include origins of replication and selectable markers permitting transformation of the host cell, e.g., the ampicillin resistance gene of E. coli and S. cerevisiae TRP1 gene, and a promoter derived from a highly-expressed gene to direct transcription of a downstream structural sequence. Such promoters can be derived from operons encoding glycolytic enzymes such as 3-phosphoglycerate kinase (PGK), α-factor, acid phosphatase, or heat shock proteins, among others. The heterologous structural sequence is assembled in appropriate phase with translation initiation and termination sequences, and preferably, a leader sequence capable of directing secretion of translated protein into the periplasmic space or extracellular medium. Optionally, the heterologous sequence can encode a fusion protein including an N-terminal identification peptide imparting desired characteristics, e.g., stabilization or simplified purification of expressed recombinant product.

Useful expression vectors for bacterial use are constructed by inserting a structural DNA sequence encoding a desired protein together with suitable translation initiation and termination signals in operable reading phase with a functional promoter. The vector will comprise one or more phenotypic selectable markers and an origin of replication to ensure maintenance of the vector and to, if desirable, provide amplification within the host. Suitable prokaryotic hosts for transformation include E. coli, Bacillus subtilis, Salmonella typhimurium and various species within the genera Pseudomonas, Streptomyces, and Staphylococcus, although others may also be employed as a matter of choice.

As a representative example, useful expression vectors for bacterial use can comprise a selectable marker and bacterial origin of replication derived from commercially available plasmids comprising genetic elements of the well known cloning vector pBR322 (ATCC 37017). Such commercial vectors include, for example, pKK223-3 (Pharmacia Fine Chemicals, Uppsala, Sweden) and GEM1 (Promega Biotec, Madison, WI, USA). These pBR322 "backbone" sections are combined with an appropriate promoter and the structural sequence to be expressed.

Following transformation of a suitable host strain and growth of the host strain to an appropriate cell density, the selected promoter is induced by appropriate means (e.g., temperature shift or chemical induction) and cells are cultured for an additional period.

Cells are typically harvested by centrifugation, disrupted by physical or chemical means, and the resulting crude extract retained for further purification.

Microbial cells employed in expression of proteins can be disrupted by any convenient method, including freeze-thaw cycling, sonication, mechanical disruption, or use of cell lysing agents, such methods are well know to those skilled in the art.

Various mammalian cell culture systems can also be employed to express recombinant protein. Examples of mammalian expression systems include the COS-7 lines of monkey kidney fibroblasts, described by Gluzman, Cell, 23:175 (1981), and other cell lines capable of expressing a compatible vector, for example, the C127, 3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences. DNA sequences derived from the SV40 splice, and polyadenylation sites may be used to provide the required nontranscribed genetic elements.

The Oxalyl-CoA Decarboxylase polypeptides can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography hydroxylapatite chromatography and lectin chromatography. Protein refolding steps can be used, as necessary, in completing configuration of the mature protein. Finally, high performance liquid chromatography (HPLC) can be employed for final purification steps.

The polypeptides of the present invention may be a naturally purified product, or a product of chemical synthetic procedures, or produced by recombinant techniques from a prokaryotic or eukaryotic host (for example, by bacterial, yeast, higher plant, insect and mammalian cells in culture). Depending upon the host employed in a recombinant production procedure, the polypeptides of the present invention may be glycosylated or may be non-glycosylated. Polypeptides of the invention may also include an initial methionine amino acid residue.

The oxalyl-CoA decarboxylase polypeptide of the present invention may be used to prevent urinary stone formation by reducing the plasma or urinary levels of the oxalate ion.

The oxalyl-CoA decarboxylase polypeptide of the present invention may also be employed to treat or prevent hyperoxaluria. Hyperoxaluria is characterized by either abnormal synthesis or hyper-absorption of oxalate which can be prevented by degrading the oxalate ions and the prevention of this disorder.

The polypeptides of the present invention are useful for identifying other molecules which have similar biological activity. An example of a screen comprises isolating the coding region of the oxalyl-CoA decarboxylase gene by using the known DNA sequence to synthesize an oligonucleotide probe. Labeled oligonucleotides having a sequence complementary to that of the gene of the present invention are used to screen a library of human cDNA, genomic DNA or mRNA to determine which members of the library the probe hybridizes to.

The Oxalyl-CoA Decarboxylase polypeptides may also be employed by expression of such polypeptides *in vivo*, which is often referred to as "gene therapy."

Thus, for example, cells from a patient may be engineered with a polynucleotide (DNA or RNA) encoding Oxalyl-CoA Decarboxylase *ex vivo*, with the engineered cells then being provided to a patient to be treated with the polypeptide. Such methods are well-known in the art. For example, cells may be engineered by procedures known in the art by use of a retroviral particle containing RNA encoding a polypeptide of the present invention.

Similarly, cells may be engineered *in vivo* for expression of a polypeptide *in vivo* by, for example, procedures known in the art. As known in the art, a producer cell for producing a retroviral particle containing RNA encoding the polypeptide of the present invention may be administered to a patient for engineering cells *in vivo* and expression of the polypeptide *in vivo.* These and other methods for administering a polypeptide of the present invention by such method should be apparent to those skilled in the art from the teachings of the present invention. For example, the expression vehicle for engineering cells may be other than a retrovirus, for example, an adenovirus which may be used to engineer cells in vivo after combination with a suitable delivery vehicle.

The polypeptides of the present invention may be employed in combination with a suitable pharmaceutical carrier. Such compositions comprise a therapeutically effective amount of the polypeptide, and a pharmaceutically acceptable carrier or excipient. Such a carrier includes but is not limited to saline, buffered saline, dextrose, water, glycerol, ethanol, and combinations thereof. The formulation should suit the mode of administration.

The pharmaceutical compositions may be administered in a convenient manner such as by the topical, intravenous, intraperitoneal, intramuscular, subcutaneous, intranasal or intradermal routes. Oxalyl-CoA decarboxylase is administered in an amount which is effective for treating and/or prophylaxis of the specific indication. In general, the oxalyl-CoA decarboxylase will be administered in an amount of at least about 10 *µ*g/kg body weight and in most cases will be administered in amounts not in excess of about 8 mg/Kg body weight per day. In most cases, the dosage is from about 10 *µ*g/kg to about 1 mg/kg body weight daily, taking into account the routes of administration, symptoms, etc.

The polypeptides, or cells expressing them can be used as an immunogen to produce antibodies thereto. These antibodies can be, for example, polyclonal or monoclonal antibodies. The present invention also includes chimeric, single chain, and humanized antibodies, as well as Fab fragments, or the product of an Fab expression library. Various procedures known in the art may be used for the production of such antibodies and fragments.

Antibodies generated against the polypeptides corresponding to a sequence of the present invention can be obtained by direct injection of the polypeptides into an animal or by administering the polypeptides to an animal, preferably a nonhuman. The antibody so obtained will then bind the polypeptides itself. In this manner, even a sequence encoding only a fragment of the polypeptides can be used to generate antibodies binding the whole native polypeptides. Such antibodies can then be used to isolate the polypeptide from tissue expressing that polypeptide.

For preparation of monoclonal antibodies, any technique which provides antibodies produced by continuous cell line cultures can be used. Examples include the hybridoma technique (Kohler and Milstein, 1975, Nature, 256:495-497), the trioma technique, the human B-cell hybridoma technique (Kozbor et al., 1983, Immunology Today 4:72), and the EBV-hybridoma technique to produce human monoclonal antibodies (Cole, et al., 1985, in Monoclonal Antibodies and Cancer Therapy, Alan R. Liss, Inc., pp. 77-96).

Techniques described for the production of single chain antibodies (U.S. Patent 4,946,778) can be adapted to produce single chain antibodies to immunogenic polypeptide products of this invention.

The present invention also relates to a diagnostic assay for detecting susceptibility to hyperoxaluria. Such an assay may be accomplished by detecting levels of oxalyl-CoA decarboxylase in a sample such as a blood or serum sample, urine, etc. The level of oxalyl-CoA decarboxylase may be detected, for example, by an immunoassay technique by procedures known in the art. An example of such an assay is a sandwich assay which utilizes two antibodies specific to oxalyl-CoA decarboxylase antigen, preferably monoclonal antibodies with one of the antibodies being labeled, eg. by coupling a suitable label such as an indicator enzyme, eg. horseradish peroxidase. The unlabeled antibody is preferably on a solid support. If antigen is present, the antigen will bind to both antibodies. After binding of the peroxidase-coupled antibody to the antigen, the peroxidase can be used to generate a colored product that is measurable and whose concentration is related to the amount of antigen in a sample. Because of the catalytic nature of the enzyme the system greatly amplifies the signal. A low level of oxalyl-CoA decarboxylase is indicative of a susceptibility to hyperoxaluria.

The present invention will be further described with reference to the following examples. All parts or amounts, unless otherwise specified, are by weight.

In order to facilitate understanding of the following examples certain frequently occurring methods and/or terms will be described.

"Plasmids" are designated by a lower case p preceded and/or followed by capital letters and/or numbers. The starting plasmids herein are either commercially available, publicly available on an unrestricted basis, or can be constructed from available plasmids in accord with published procedures. In addition, equivalent plasmids to those described are known in the art and will be apparent to the ordinarily skilled artisan.

"Digestion" of DNA refers to catalytic cleavage of the DNA with a restriction enzyme that acts only at certain sequences in the DNA. The various restriction enzymes used herein are commercially available and their reaction conditions, cofactors and other requirements were used as would be known to the ordinarily skilled artisan. For analytical purposes, typically 1 *µ*g of plasmid or DNA fragment is used with about 2 units of enzyme in about 20 *µ*l of buffer solution. For the purpose of isolating DNA fragments for plasmid construction, typically 5 to 50 *µ*g of DNA are digested with 20 to 250 units of enzyme in a larger volume. Appropriate buffers and substrate amounts for particular restriction enzymes are specified by the manufacturer. Incubation times of about 1 hour at 37°C are ordinarily used, but may vary in accordance with the supplier's instructions. After digestion the reaction is electrophoresed directly on a polyacrylamide gel to isolate the desired fragment.

Size separation of the cleaved fragments is performed using 8 percent polyacrylamide gel described by Goeddel, D. *et al*., Nucleic Acids Res., 8:4057 (1980).

"Oligonucleotides" refers to either a single stranded polydeoxynucleotide or two complementary polydeoxynucleotide strands which may be chemically synthesized. Such synthetic oligonucleotides have no 5' phosphate and thus will not ligate to another oligonucleotide without adding a phosphate with an ATP in the presence of a kinase. A synthetic oligonucleotide will ligate to a fragment that has not been dephosphorylated.

"Ligation" refers to the process of forming phosphodiester bonds between two double stranded nucleic acid fragments (Maniatis, T., et al., Id., p. 146). Unless otherwise provided, ligation may be accomplished using known buffers and conditions with 10 units to T4 DNA ligase ("ligase") per 0.5 *µ*g of approximately equimolar amounts of the DNA fragments to be ligated.

Unless otherwise stated, transformation was performed as described in the method of Graham, F. and Van der Eb, A., Virology, 52:456-457 (1973).

### Example 1

### Bacterial Expression and Purification of Oxalyl-CoA decarboxylase

The DNA sequence encoding for oxalyl-CoA decarboxylase, ATCC # 75715, is initially amplified using PCR oligonucleotide primers corresponding to the 5' and 3' sequences of the processed oxalyl-CoA decarboxylase protein (minus the signal peptide sequence) and additional nucleotides corresponding to Nco I and Bgl II were added to the 5' and 3' sequences respectively. The primers used for generation of the PCR fragment will encode the OmpA leader sequence in addition to the restriction sites in the sequence encoding the human oxalyl-CoA decarboxylase. The 5' oligonucleotide primer has the sequence 5'-GACTTCATGAAAAAGACAGATATCGCAATTGCAGTGGCACTGGCTGGTTTCGCTACCGT TGCGCAAGCTGCTCCGGACAGTAACTTCGCAGAG - 3', contains a BspH I restriction enzyme site followed by 21 nucleotides of the human oxalyl-CoA decarboxylase gene; the 3' sequence is 3'-CAGTTCTAGACATATTAGAGCGGGTCAGCC - 5', contains complementary sequences to Bgl II restriction enzyme site, a translation stop codon and the last 20 nucleotides of the human oxalyl-CoA decarboxylase coding sequence. The restriction enzyme sites correspond to the restriction enzyme sites on the bacterial expression vector pQE-60 (Qiagen, Inc. 9259 Eton Ave., Chatsworth, CA 91311). pQE-60 encodes antibiotic resistance (Amp^{r}), a bacterial origin of replication (ori), an IPTG-regulatable promoter operator (P/O), a ribosome binding site (RBS), a 6-His tag and restriction enzyme sites. pQE-60 was then digested with Nco I and Bgl II. The amplified sequences were ligated into PQE-60 and were inserted in frame with the sequence encoding for the histidine tag and the RBS. The ligation mixture was then used to transform E. coli strain M15/rep4 available from Qiagen under the trademark M15/rep 4. M15/rep4 contains multiple copies of the plasmid pREP4, which expresses the lacI repressor and also confers kanamycin resistance (Kan^{r}). Transformants are identified by their ability to grow on LB plates and ampicillin/kanamycin resistant colonies were selected. Plasmid DNA was isolated and confirmed by restriction analysis. Clones containing the desired constructs were grown overnight (O/N) in liquid culture in LB media supplemented with both Amp (100 ug/ml) and Kan (25 ug/ml). Tho O/N culture is used to inoculate a large culture at a ratio of 1:100 to 1:250. The cells were grown to an optical density 600 (O.D.⁶⁰⁰) of between 0.4 and 0.6. IPTG (Isopropyl-B-D-thiogalacto pyranoside) was then added to a final concentration of 1 mM. IPTG induces by inactivating the lacI repressor, clearing the P/O leading to increased gene expression. Cells were grown an extra 3 to 4 hours. Cells were then harvested by centrifugation (20 mins at 6000Xg). The cell pellet was solubilized in the chaotropic agent 6 Molar Guanidine HCl. After clarification, solubilized oxalyl-CoA decarboxylase was purified from this solution by chromatography on a Nickel-Chelate column under conditions that allow for tight binding by proteins containing the 6-His tag. (Hochuli, E. et al., Genetic Engineering, Principles & Methods, 12:87-98 (1990). Protein renaturation out of GnHCl can be accomplished by several protocols. (Jaenicke, R. and Rudolph, R., Protein Structure - A Practical Approach, IRL Press, New York (1990)). Oxalyl-CoA decarboxylase (95% pure) was eluted from the column in 6 molar guanidine HCl pH 5.0 and for the purpose of renaturation adjusted to 3 molar guanidine HCl, 100mM sodium phosphate, 10 mmolar glutathione (reduced) and 2 mmolar glutathione (oxidized).

### Example 2

### Expression of Human Oxalyl-CoA Decarboxylase by in vitro transcription and translation

The *in vitro* transcription and translation of the oxalyl-CoA decarboxylase was carried out using the TNT Coupled Reticulocyte Lysate System (Promega, Madison, WI). The cDNA encoding for oxalyl-CoA decarboxylase was cloned directionally EcoRI to XhoI with the EcoRI site defining the 5' end of the gene and the XhoI site defining the 3' end of the gene. The gene was inserted in the T3 direction. T3 defines a bacteriophage RNA polymerase which recognizes a specific promoter, and transcribes the DNA into a mRNA. A rabbit reticulocyte lysate is supplemented with T3 RNA polymerase and directs the expression of proteins with a T3 promoter utilizing the T3 RNA polymerase to transcribe the message, and the reticulocyte lysate to translate the nascent RNA. By incorporating radioactive amino acids into the translated product, protein expression can be analyzed using SDS-polyacrylamide gel electrophoresis followed by autoradiography. More specifically, 1*µ*g of plasmid containing the oxalyl-CoA decarboxylase DNA was incubated at 30°C for 1 hour with the reticulocyte lysate, T3 RNA polymerase and [³⁵S]-Methionine. After incubation, the translations were analyzed by SDS-PAGE and autoradiography. A prominent translation product was visible at ≈55Kd.

### SEQUENCE LISTING

(1) GENERAL INFORMATION:
   (i) APPLICANT: OLSEN, ET AL.
   (ii) TITLE OF INVENTION: Human Oxalyl-CoA Decarboxylase
   (iii) NUMBER OF SEQUENCES: 2
   (iv) CORRESPONDENCE ADDRESS:
      (A) ADDRESSEE: CARELLA, BYRNE, BAIN, GILFILLAN, CECCHI, STEWART & OLSTEIN
      (B) STREET: 6 BECKER FARM ROAD
      (C) CITY: ROSELAND
      (D) STATE: NEW JERSEY
      (E) COUNTRY: USA
      (F) ZIP: 07068
   (v) COMPUTER READABLE FORM:
      (A) MEDIUM TYPE: 3.5 INCH DISKETTE
      (B) COMPUTER: IBM PS/2
      (C) OPERATING SYSTEM: MS-DOS
      (D) SOFTWARE: WORD PERFECT 5.1
   (vi) CURRENT APPLICATION DATA:
      (A) APPLICATION NUMBER:
      (B) FILING DATE: SUBMITTED HEREWITH
      (C) CLASSIFICATION:
   (vii) PRIOR APPLICATION DATA
      (A) APPLICATION NUMBER:
      (B) FILING DATE:
   (viii) ATTORNEY/AGENT INFORMATION:
      (A) NAME: FERRARO, GREGORY D.
      (B) REGISTRATION NUMBER: 36,134
      (C) REFERENCE/DOCKET NUMBER: 325800-80
   (ix) TELECOMMUNICATION INFORMATION:
      (A) TELEPHONE: 201-994-1700
      (B) TELEFAX: 201-994-1744
(2) INFORMATION FOR SEQ ID NO:1:
   (i) SEQUENCE CHARACTERISTICS
      (A) LENGTH: 1,882 BASE PAIRS
      (B) TYPE: NUCLEIC ACID
      (C) STRANDEDNESS: SINGLE
      (D) TOPOLOGY: LINEAR
   (ii) MOLECULE TYPE: cDNA
   (xi) SEQUENCE DESCRIPTION: SEQ ID NO:1:
(2) INFORMATION FOR SEQ ID NO:2:
   (i) SEQUENCE CHARACTERISTICS
      (A) LENGTH: 578 AMINO ACIDS
      (B) TYPE: AMINO ACID
      (C) STRANDEDNESS:
      (D) TOPOLOGY: LINEAR
   (ii) MOLECULE TYPE: PROTEIN
   (xi) SEQUENCE DESCRIPTION: SEQ ID NO:2:

## Claims

1. A polynucleotide selected from the group consisting of
(a) polynucleotides encoding the mature form of the polypeptide having the deduced amino acid sequence as shown in Figure 1;
(b) polynucleotides having the coding sequence as shown in Figure 1 encoding the mature form of the polypeptide;
(c) polynucleotides encoding the polypeptide having the amino acid sequence of the mature form of the polypeptide encoded by the cDNA contained in ATCC 75715;
(d) polynucleotides having the coding sequence of the cDNA contained in ATCC 75715 encoding the mature form of the polypeptide;
(e) polynucleotides encoding a polypeptide encoded by a polynucleotide of any one of (a) to (d) except the N-terminal methionine;
(f) polynucleotides encoding a polypeptide encoded by a polynucleotide of any one of (a) to (d) in which one or more amino acid residues are substituted with a conserved amino acid residue, and which encode a polypeptide having Oxalyl-CoA decarboxylase activity;
(g) polynucleotides of any one of (a) to (d) wherein one or more nucleotides are deleted or added and which encode a polypeptide having oxalyl-CoA decarboxylase activity;
(h) polynucleotides encoding an antigenic fragment of a polypeptide encoded by a polynucleotide of any one of (a) to (d); and
(i) polynucleotides the complementary strand of which hybridizes with and is at least 70% identical to a polynucleotide as defined in any one of (a) to (d) and which encode a polypeptide having oxalyl-CoA decarboxylase activity;
or the complementary strand of a polynucleotide of any one of (a) to (i).

2. The polynucleotide of claim 1 which is DNA.

3. The DNA of claim 2 which is genomic DNA.

4. The polynucleotide of claim 1 which is RNA.

5. A vector containing the polynucleotide of any one of claims 1 to 4.

6. The vector of claim 5 in which the polynucleotide is operatively linked to expression control sequences allowing expression in prokaryotic or eukaryotic host cells.

7. A host cell genetically engineered with the vector of claim 5 or 6.

8. A process for producing an oxalyl-CoA decarboxylase comprising: culturing the host cell of claim 7 and recovering the polypeptide encoded by said polynucleotide from the culture.

9. A process for producing cells capable of expressing an oxalyl-CoA decarboxylase comprising genetically engineering cells with the vector of claim 5 or 6.

10. A polypeptide encoded by a polynucleotide of any one of claims 1 to 4 or obtainable by the process of claim 8.

11. An antibody against the polypeptide of claim 10.

12. A nucleic acid molecule which hybridizes to a polynucleotide of any one of claims 1 to 4 under stringent hybridization conditions.

13. A pharmaceutical composition comprising the polynucleotide of any one of claims 1 to 4 or the polypeptide of claim 10 and optionally a pharmaceutically acceptable carrier.

14. A diagnostic composition comprising the polynucleotide of any one of claims 1 to 4, the nucleic acid molecule of claim 12 or the antibody of claim 11.

15. Use of an polypeptide of claim 10 or of a polynucleotide of any one of claims 1 to 4 for the preparation of a pharmaceutical composition for the prevention of urinary stone formation or for the treatment and/or prevention of hyperoxaluria.

16. A method for detecting a susceptibility to hyperoxaluria comprising:
(a) detecting in a sample the amount of the polypeptide of claim 10; or
(b) identifying the concentration of oxalyl-CoA decarboxylase in the sample; and
(c) determining whether the host has a susceptibility to hyperoxaluria.

## Patentansprüche

1. Polynucleotid, ausgewählt aus folgender Gruppe:
(a) Polynucleotide, die die reife Form des Polypeptids codieren, das die in Figur 1 gezeigte abgeleitete Aminosäuresequenz besitzt;
(b) Polynucleotide, welche die codierende Sequenz, wie in Figur 1 dargestellt, besitzen, die die reife Form des Polypeptids codiert;
(c) Polynucleotide, welche das Polypeptid codieren, das die Aminosäuresequenz der reifen Form des Polypeptids besitzt, das von der cDNA, die in ATCC 75715 enthalten ist, codiert wird;
(d) Polynucleotide, welche die codierende Sequenz der cDNA besitzen, die in ATCC 75715 enthalten ist, die die reife Form des Polypeptids codiert;
(e) Polynucleotide, die ein Polypeptid codieren, das von einem Polynucleotid nach einer der Definitionen (a) bis (d) codiert wird, ausgenommen das N-terminale Methionin;
(f) Polynucleotide, welche ein Polypeptid codieren, das von Polynucleotiden nach einer der Definitionen (a) bis (d) codiert wird, in dem ein oder mehrere Aminosäurereste durch konservierte Aminosäurereste ersetzt sind und die ein Polypeptid mit Oxalyl-CoA-Decarboxylase-Aktivität codieren;
(g) Polynucleotide, nach einer der Definitionen (a) bis (d), wobei eines oder mehrere Nucleotide deletiert oder hinzugefügt werden, und die ein Polypeptid mit Oxalyl-CoA-Decarboxylase-Aktivität codieren;
(h) Polynucleotide, welche ein antigenes Fragment eines Polypeptids codieren, welches von einem Polynucleotid nach einer der Definitionen (a) bis (d) codiert wird; und
(i) Polynucleotide, deren komplementärer Strang hybridisiert und zu mindestens 70 % identisch ist mit einem Polynucleotid, wie in einer der Definitionen (a) bis (d) definiert, und die ein Polypeptid mit Oxalyl-CoA-Decarboxylase-Aktivität codieren;
oder der komplementäre Strang eines Polynucleotids nach einer der Definitionen (a) bis (i).

2. Polynucleotid nach Anspruch 1, welches DNA ist.

3. DNA nach Anspruch 2, welche genomische DNA ist.

4. Polynucleotid nach Anspruch 1, welches RNA ist.

5. Vektor, welcher das Polynucleotid nach einem der Ansprüche 1 bis 4 enthält.

6. Vektor nach Anspruch 5, in welchem das Polynucleotid funktionell mit den Expression kontrollierenden Sequenzen verknüpft ist, die die Expression in prokaryontischen oder eukaryontischen Wirtszellen erlauben.

7. Wirtszelle, die mit dem Vektor nach Anspruch 5 oder 6 genetisch verändert wurde.

8. Verfahren zur Herstellung einer Oxalyl-CoA-Decarboxylase, umfassend die Züchtung der Wirtszelle nach Anspruch 7 und die Gewinnung des Polypeptids, welches von dem Polynucleotid codiert wird, aus der Kultur.

9. Verfahren zur Produktion von Zellen, welche in der Lage sind, eine Oxalyl-CoA-Decarboxylase zu exprimieren, umfassend die genetische Veränderung von Zellen mit dem Vektor nach Anspruch 5 oder 6.

10. Polypeptid, welches von einem Polynucleotid nach einem der Ansprüche 1 bis 4 codiert wird oder gemäß dem Verfahren nach Anspruch 8 erhältlich ist.

11. Antikörper gegen das Polypeptid nach Anspruch 10.

12. Nucleinsäuremolekül, welches mit einem Polynucleotid nach einem der Ansprüche 1 bis 4 unter stringenten Hybridisierungsbedingungen hybridisiert.

13. Arzneimittel, umfassend das Polynucleotid nach einem der Ansprüche 1 bis 4 oder das Polypeptid nach Anspruch 10 und gegebenenfalls einen pharmazeutisch verträglichen Träger.

14. Diagnosemittel, umfassend das Polynucleotid nach einem der Ansprüche 1 bis 4, das Nucleinsäuremolekül nach Anspruch 12 oder den Antikörper nach Anspruch 11.

15. Verwendung eines Polypeptids nach Anspruch 10 oder eines Polynucleotids nach einem der Ansprüche 1 bis 4 für die Herstellung eines Arzneimittels zur Vorbeugung gegen Harnsteinbildung oder zur Behandlung und/oder Vermeidung von Hyperoxalurie.

16. Verfahren zum Nachweis einer Anfälligkeit für Hyperoxalurie, umfassend:
(a) Nachweis der Menge des Polypeptids nach Anspruch 10 in einer Probe; oder
(b) Identifizierung der Konzentration von Oxalyl-CoA-Decarboxylase in der Probe; und
(c) Bestimmung, ob der Wirt eine Anfälligkeit für Hyperoxalurie hat.

## Revendications

1. Polynucléotide choisi dans le groupe consistant en :
(a) polynucléotides codant pour la forme mature du polypeptide ayant la séquence d'acides aminés déduite telle que représentée à la figure 1 ;
(b) polynucléotides ayant la séquence codante telle que représentée à la figure 1 codant pour la forme mature du polypeptide;
(c) polynucléotides codant pour le polypeptide ayant la séquence d'acides aminés de la forme mature du polypeptide codé par l'ADNc contenue dans ATTCC 75715 ;
(d) polynucléotides ayant la séquence codante de l'ADNc contenue dans ATTCC 75715 codant pour la forme mature du polypeptide ;
(e) polynucléotides codant pour un polypeptide codé par un polynucléotide de l'un de (a) à (d) à l'exception de la méthionine N-terminale ;
(f) polynucléotides codant pour un polypeptide codé par un polynucléotide selon l'un de (a) à (d) dans lequel un ou plusieurs restes d'acides aminés sont remplacés par un reste d'acide aminé conservé, et qui code pour un polypeptide ayant une activité oxalyl-CoA décarboxylase ;
(g) polynucléotides selon l'un de (a) à (d) dans lesquels l'un ou plusieurs nucléotides sont délétés ou ajoutés et qui codent pour un polypeptide ayant une activité oxalyl-CoA décarboxylase ;
(h) polynucléotides codant pour un fragment antigénique d'un polypeptide codé par un polynucléotide selon l'un de (a) à (d) ; et
(i) polynucléotides dont le brin complémentaire hybride avec et est au moins à 70 % identique à un polynucléotide tel que défini dans l'un de (a) à (d) et qui codent pour un polypeptide ayant une activité oxalyl-CoA décarboxylase ;
ou le brin complémentaire d'un polynucléotide selon l'un de (a) à (i).

2. Polynucléotide selon la revendication 1 qui est de l'ADN.

3. ADN selon la revendication 2 qui est de l'ADN génomique.

4. Polynucléotide selon la revendication 1 qui est de l'ARN.

5. Vecteur contenant le polynucléotide selon l'une quelconque des revendications 1 à 4.

6. Vecteur selon la revendication 5 dans lequel le polynucléotide est lié de manière opérationnelle à des séquences de contrôle d'expression permettant l'expression dans des cellules hôtes procaryotes ou eucaryotes.

7. Cellule hôte obtenue par génie génétique au moyen du vecteur de la revendication 5 ou 6.

8. Procédé de production d'une oxalyl-CoA décarboxylase comprenant la culture de la cellule hôte selon la revendication 7 et la récupération du polypeptide codant pour ledit polynucléotide à partir de la culture.

9. Procédé de production de cellules capables d'exprimer une oxalyl-CoA décarboxylase comprenant l'obtention par génie génétique de cellules au moyen du vecteur de la revendication 5 ou 6.

10. Polypeptide codé par un polynucléotide selon l'une quelconque des revendications 1 à 4 ou pouvant être obtenu par le procédé de la revendication 8.

11. Anticorps contre le polypeptide de la revendication 10.

12. Molécule d'acide nucléique qui hybride avec un polynucléotide selon l'une quelconque des revendications 1 à 4 dans des conditions d'hybridation stringentes.

13. Composition pharmaceutique comprenant le polynucléotide selon l'une quelconque des revendications 1 à 4 ou le polypeptide de la revendication 10 et éventuellement un support pharmaceutiquement acceptable.

14. Composition de diagnostic comprenant le polynucléotide selon l'une quelconque des revendications 1 à 4, la molécule d'acide nucléique de la revendication 12 ou l'anticorps de la revendication 11.

15. Utilisation d'un polypeptide de la revendication 10 ou d'un polynucléotide selon l'une quelconque des revendications 1 à 4 pour la préparation d'une composition pharmaceutique pour la prévention de la formation de calculs rénaux ou pour le traitement et/ou la prévention de l'hyperoxalurie.

16. Méthode de détection d'une sensibilité à l'hyperoxalurie comprenant les étapes consistant à :
(a) détecter dans un échantillon une quantité du polynucléotide de la revendication 10 ; ou
(b) identifier la concentration d'oxalyl-CoA décarboxylase dans l'échantillon ; et
(c) déterminer si l'hôte a une sensibilité vis à vis de l'hyperoxalurie.
